# EUROPEAN PATENT APPLICATION

(11) **EP 4 834 275 A1**
(43) Date of publication of application: **04.11.2026**
(21) Application number: 24913011.3
(22) Date of filing: 26.12.2024
(51) Int. Cl.: A61C 13/083, A61C 13/08, A61K 6/816, A61K 6/818, A61K 6/822, A61K 6/824, C01G 25/00, C01G 33/00, C04B 35/488, C04B 41/86

(54) **METHOD FOR MANUFACTURING DENTAL PROSTHESIS**

(30) Priority: 28.12.2023 JP 2023222899
(71) Applicant: Kuraray Noritake Dental Inc., Kurashiki-shi, Okayama 710-0801 (JP)
(72) Inventor: TAJIMA Yuta, Miyoshi-shi, Aichi 470-0293 (JP); NAKANO Kirihiro, Miyoshi-shi, Aichi 470-0293 (JP); NIWA Takahiro, Miyoshi-shi, Aichi 470-0293 (JP); KATO Shinichiro, Miyoshi-shi, Aichi 470-0293 (JP)
(74) Representative: D Young & Co LLP
(86) International application number: PCT/JP2024/046165
(87) International publication number: WO 2025/143125

(57) **Abstract**

The present invention provides a method for producing a dental prosthesis whereby defective fusing of porcelain is reduced using a zirconia composite sintered body comprising Nb₂O₅ and/or Ta₂O₅. The present invention relates to a method for producing a dental prosthesis comprising the steps of: subjecting a zirconia composite sintered body comprising Nb₂O₅ and/or Ta₂O₅ to heat treatment or polishing as a pretreatment; and building up porcelain on the zirconia composite sintered body subjected to the pretreatment, followed by firing.

## Description

### TECHNICAL FIELD

The present invention relates to a method for producing a dental prosthesis. More specifically, the present invention relates to a method for producing a dental prosthesis whereby defective fusing of porcelain is reduced using a zirconia composite sintered body comprising Nb₂O₅ or Ta₂O₅.

### BACKGROUND ART

Traditionally, metals have been commonly used for dental products (for example, prostheses such as veneer crowns, dental caps or crowns, and post crowns). However, metals have the drawback of lacking aesthetics, and occasionally cause allergic reactions due to leaching of metal.

To address the issues associated with the use of metals, ceramic materials such as aluminum oxide (alumina) or zirconium oxide (zirconia) have been used for dental products as alternatives to metal. Zirconia sintered bodies, in particular, offer superior aesthetics and strength, and have seen a rise in demand, especially due to their increasing affordability in recent years.

To achieve improved aesthetics in the oral cavity, it is necessary for dental products to mimic the look of natural teeth.

However, achieving a natural tooth-like appearance, especially in terms of transparency, gloss (glaze or shine), and color tone, can be difficult with a zirconia sintered body alone.

This issue is addressed by a veneer crown aiming to replicate the appearance of natural teeth by fusing a ceramic material called porcelain (for example, Patent Literature 1) onto an exposed surface of a frame (substrate) formed of a zirconia sintered body, rather than leaving the zirconia sintered body exposed. Such dental products are called porcelain-fused-to-zirconia (PFZ) crowns.

It has been known that, when building up porcelain on a zirconia sintered body, the porcelain can more easily fuse onto the zirconia sintered body and become more evenly distributed on the substrate when the zirconia sintered body, serving as the substrate, is sandblasted to form surface irregularities and increase wettability before building up porcelain, as indicated in Patent Literature 1.

It has also been known to build up porcelain directly on a zirconia sintered body serving as the substrate, without carrying out any treatment on the zirconia sintered body at the time of porcelain build-up.

Once fully sintered, zirconia sintered bodies exhibit hardness that makes it nearly impossible to process with a dental processing machine. For example, machining a cubic zirconia sintered body into the desired tooth form for a patient results in substantial wear on metal processing tools and demands a considerable amount of time, even for producing just one dental prosthesis.

For these reasons, zirconia sintered bodies, when used in dental material applications, are typically processed into the shape of a desired dental prosthesis while in a more easily processable, semi-sintered state known as a pre-sintered body, rather than as a fully sintered body, before it is sintered into a sintered body in the required dental prosthesis shape. Afterward, minor adjustments are made to the sintered body to ensure that its shape as a dental prosthesis fits comfortably when placed in the patient's mouth at the dental clinic.

As a result, the entire treatment often takes more than a month to complete from the start of treatment at the dental clinic.

If extensive machining of a zirconia sintered body is possible in the sintered state, it can significantly shorten the entire treatment period from the start of treatment.

Under these circumstances, there have been proposed zirconia sintered bodies that exhibit superior machinability in the sintered state and can be processed into a desired dental prosthesis shape from prism- or disc-shaped mill blanks (for example, such as in Patent Literature 2).

Patent Literature 2 discloses a processable zirconia as a sintered body formed by incorporating a tetragonal zirconia composite powder and a TiO₂ nanopowder, where the tetragonal zirconia composite powder comprises 79.8 to 92 mol% ZrO₂ and 4.5 to 10.2 mol% Y₂O₃ along with 3.5 to 7.5 mol% Nb₂O₅ or 5.5 to 10.0 mol% Ta₂O₅, and the TiO₂ nanopowder is incorporated in a mass ratio of more than 0 mass% and 2.5 mass% or less relative to the zirconia composite powder. A method of production of this zirconia sintered body is also disclosed.

### CITATION LIST

### Patent Literature

Patent Literature 1: JP2017-122064 A
Patent Literature 2: JP2015-127294 A

### SUMMARY OF INVENTION

### Technical Problem

However, the present inventors have found that porcelain fails to fuse onto the substrate when a zirconia sintered body having the composition disclosed in Patent Literature 2 is used as the substrate, regardless of whether porcelain is built up on the substrate after sandblasting the zirconia sintered body as in Patent Literature 1, or porcelain is directly built up on the substrate without performing any treatment on the zirconia sintered body. This problem was found to be particularly pronounced in so-called "glaze firing", which is performed as a finishing step in prosthesis fabrication by building up a thin porcelain layer on the substrate (for example, either by coating or spraying a spray-type porcelain) before firing.

It is an object of the present invention to provide a method for producing a dental prosthesis whereby defective fusing of porcelain is reduced using a zirconia composite sintered body comprising Nb₂O₅ and/or Ta₂O₅.

### Solution to Problem

The present inventors conducted intensive studies to find a solution to the foregoing problem, and found that it can be solved with heat treatment or polishing performed on a zirconia composite sintered body comprising Nb₂O₅ and/or Ta₂O₅. Building upon this finding, the present inventors conducted additional examinations, ultimately resulting in the completion of the present invention.

Specifically, the present invention includes the following.
[1] A method for producing a dental prosthesis, comprising the steps of:
   subjecting a zirconia composite sintered body comprising Nb₂O₅ and/or Ta₂O₅ to heat treatment or polishing as a pretreatment; and
   building up porcelain on the zirconia composite sintered body subjected to the pretreatment, followed by firing.
[2] The method for producing a dental prosthesis according to [1], wherein the heat treatment is carried out at a temperature of 150°C or more and 1,550°C or less.
[3] The method for producing a dental prosthesis according to [1], wherein the zirconia composite sintered body has a surface roughness Ra of 8.0 µm or less after being polished in the polishing step.
[4] The method for producing a dental prosthesis according to [1] or [2], wherein the zirconia composite sintered body comprises zirconia, and a stabilizer capable of preventing a phase transformation of zirconia, and
   the zirconia content is 78 to 97.5 mol%, the content of the stabilizer is 1 to 12 mol%, and the content of Nb₂O₅ and/or Ta₂O₅ is 1 to 9 mol% in total 100 mol% of zirconia, the stabilizer, Nb₂O₅, and Ta₂O₅.
[5] The method for producing a dental prosthesis according to [4], wherein the zirconia composite sintered body further comprises an element or ion derived from a capping agent.
[6] The method for producing a dental prosthesis according to [5], wherein the content of the element or ion derived from a capping agent is more than 0 mol% and 5 mol% or less relative to total 100 mol% of zirconia, the stabilizer, Nb₂O₅, and Ta₂O₅.
[7] The method for producing a dental prosthesis according to [5] or [6], wherein the element or ion derived from a capping agent belongs to periods 2 to 7 of the periodic table, and is an element or an ion thereof having a first ionization energy lower than that of a group 18 element of the same period, and/or an element or an ion thereof having a high electron affinity.
[8] The method for producing a dental prosthesis according to [5] or [6], wherein the element or ion derived from a capping agent comprises at least one element or ion thereof selected from the group consisting of Cu, Ag, Li, Na, K, Rb, Cs, Fr, At, I, Br, Cl, and F.
[9] The method for producing a dental prosthesis according to [5] or [6], wherein the element or ion derived from a capping agent comprises at least one element or ion thereof selected from the group consisting of Li, Na, K, Rb, Cs, and Fr.
[10] The method for producing a dental prosthesis according to any one of [4] to [9], wherein the zirconia composite sintered body has a ratio A/B of 0.9 or more and 3 or less, where A is the content of the stabilizer in mol%, and B is the combined content of Nb₂O₅ and Ta₂O₅ in mol%.
[11] The method for producing a dental prosthesis according to any one of [4] to [10], wherein the stabilizer comprises Y₂O₃ and/or CeO₂.
[12] The method for producing a dental prosthesis according to any one of [4] to [11], wherein the zirconia composite sintered body further comprises a zirconia enhancer, and the content of the zirconia enhancer is more than 0 mass% and 6.0 mass% or less relative to total 100 mass% of zirconia, the stabilizer, and Nb₂O₅ and Ta₂O₅.
[13] The method for producing a dental prosthesis according to [12], wherein the zirconia enhancer comprises TiO₂ and/or Al₂O₃.
[14] The method for producing a dental prosthesis according to [13], wherein the zirconia enhancer comprises TiO₂, and the content of TiO₂ is 0.6 to 4.5 mass%.
[15] The method for producing a dental prosthesis according to any one of [1] to [14],
wherein the zirconia composite sintered body has an average crystal grain size of 0.5 to 5.0 µm.

### Advantageous Effects of Invention

According to the present invention, a method for producing a dental prosthesis can be provided whereby defective fusing of porcelain is reduced using a zirconia composite sintered body comprising Nb₂O₅ and/or Ta₂O₅.

### BRIEF DESCRIPTION OF DRAWINGS

[FIG. 1] FIG. 1 shows photographs illustrating the frontal and occlusal aspects of an anterior teeth, a premolar, and a molar.
[FIG. 2] FIG. 2 shows photographs representing the result of evaluating the glaze firing of a dental prosthesis obtained in Example 1 of the present invention.
[FIG. 3] FIG. 3 shows photographs representing the result of evaluating the glaze firing of a dental prosthesis obtained in Comparative Example 1 of the present invention.

### DESCRIPTION OF EMBODIMENTS

A method for producing a dental prosthesis of the present invention comprises the steps of:
subjecting a zirconia composite sintered body comprising Nb₂O₅ and/or Ta₂O₅ to heat treatment or polishing as a pretreatment; and
building up porcelain on the zirconia composite sintered body subjected to the pretreatment, followed by firing.

As used herein, "molded body" refers to a state in which neither a semi-sintered state (pre-sintered state) nor a sintered state has been reached. That is, "molded body" distinguishes itself from pre-sintered body and sintered body in that it remains unfired after being molded.

As used herein, "zirconia composite pre-sintered body" refers to a semi-sintered state in which a raw material powder such as zirconia has formed necks but has not fully sintered.

As used herein, "zirconia composite sintered body" refers to a sintered state in which a raw material powder such as zirconia has fully sintered. In a zirconia composite sintered body, sintering has caused a raw material powder such as zirconia to consolidate or progressively form a solid solution, increasing the relative density and promoting densification, resulting in a fully sintered state with a relative density of 95% or more.

As used herein, "zirconia" refers to zirconium(IV) oxide (ZrO₂), with a trace amount (0.5 mass% or more and 3 mass% or less) of HfO₂ relative to the amount of ZrO₂. Because HfO₂ is difficult to separate, terms such as "zirconia" and "zirconia powder" are intended to include both ZrO₂ and HfO₂. A powder with a stabilizer present in zirconia as a solid solution is also encompassed by "zirconia powder."

As used herein, "in the atmosphere" means under standard atmospheric pressure (1 atm).

As used herein, "zirconia enhancer" refers to a component that serves to enhance the mechanical strength of the zirconia composite sintered body.

As used herein, the term "build up" refers to laying porcelain on a substrate using a tool such as a brush, and encompasses not only coating porcelain on a substrate but also spraying a spray-type porcelain on a substrate.

The content of zirconia enhancer (mass%) refers to the proportion external to total 100 mass% of zirconia, the stabilizer, Nb₂O₅, and Ta₂O₅. Accordingly, the content of the zirconia enhancer in the zirconia composite sintered body can be calculated from the amount (mass) of raw material charged.

In this specification, the content of each component in the zirconia composite sintered body can be calculated from the amounts of raw materials charged.

In this specification, machining encompasses both cutting and grinding. Machining may be a wet or dry process, without specific restrictions.

As used herein, "surface roughness Ra" refers to an arithmetic mean roughness according to JIS B 0601:2013.

In this specification, the upper limits and lower limits of numeric ranges (including, for example, temperature ranges, the content of each component, the abundance of crystal systems, values calculated from components, and various physical properties) can be appropriately combined.

It is not entirely clear as to why a method for producing a dental prosthesis of the present invention can reduce defective fusing of porcelain on a zirconia composite sintered body serving as the substrate. However, the following speculation has been made.

The zirconia composite sintered body, serving as the substrate, comprises Nb₂O₅ and/or Ta₂O₅, and, unlike a conventional zirconia sintered body obtained by machining a pre-sintered body, treating the zirconia composite sintered body by heat treatment or polishing appears to alter the surface condition of the substrate, allowing porcelain to more readily fuse onto the substrate surface (increasing the chemical affinity between the substrate surface and porcelain).

As a more specific example, a substrate comprising Nb₂O₅ and/or Ta₂O₅, when subjected to heat treatment or polishing, appears to result in dehydration of hydrophilic groups generated on the substrate surface through wet machining, rendering the surface hydrophobic and thereby reducing defective fusing of porcelain.

In view of facilitating reduction of defective porcelain fusing, the heat treatment temperature in a method for producing a dental prosthesis of the present invention is preferably 150°C or more and 1,550°C or less.

Compared with anterior teeth, which are more planar and simpler in shape, premolars have a greater number of curved surfaces and more complex shapes, such as fossae and grooves, as seen in the occlusal aspect. This makes it more difficult to reduce defective fusing of porcelain on premolars.

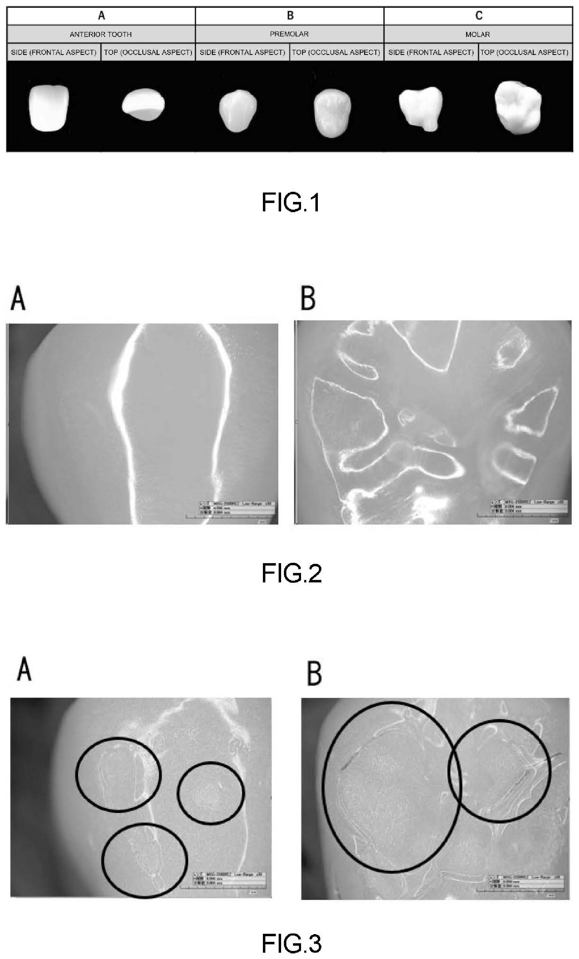

Similar to premolars, molars also have multiple curved surfaces, with fossae and grooves that are even more complex in shape than those observed in premolars in the occlusal aspect, as shown in FIG. 1. Furthermore, the greater number of cusp tips adds more complexity to the shape of molars, increasing the vertical distance between the cusp tips and the fossae and grooves. This makes it even more difficult to reduce defective fusing of porcelain on molars.

In view of facilitating reduction of defective porcelain fusing irrespective of the shape of the dental prosthesis even with regions complex in shape and including a number of curved surfaces along with multiple grooves seen in the occlusal aspect, the heat treatment temperature is more preferably 180°C or more, even more preferably 200°C or more, particularly preferably 250°C or more.

In view of facilitating reduction of defective porcelain fusing irrespective of the shape of the dental prosthesis even with regions complex in shape and including a number of curved surfaces along with multiple grooves seen in the occlusal aspect, the heat treatment temperature is more preferably 1,500°C or less, even more preferably 1,450°C or less, particularly preferably 1,400°C or less.

In view of facilitating reduction of defective porcelain fusing, the duration of the heat treatment in a method for producing a dental prosthesis of the present invention is preferably 10 seconds or more, more preferably 30 seconds or more. In view of facilitating reduction of defective porcelain fusing irrespective of the shape of the dental prosthesis even with regions complex in shape and including a number of curved surfaces along with multiple grooves seen in the occlusal aspect, the duration of heat treatment is even more preferably 50 seconds or more, particularly preferably 1 minute or more.

The upper limit of heat treatment time is not particularly limited, as long as the present invention can exhibit its effects. However, considering time constraints during actual procedures in clinical settings, the duration of heat treatment is preferably 120 minutes or less, more preferably 60 minutes or less, even more preferably 30 minutes or less, particularly preferably 10 minutes or less.

The heating device employed for the heat treatment is not particularly limited and a known heating device may be used (for example, such as a furnace), as long as the temperature and the duration of the heat treatment can be adjusted to the foregoing ranges. The heat treatment may be carried out in a vacuum. It may be possible to set a lower temperature for the heat treatment when it is performed in a vacuum.

In view of facilitating reduction of defective porcelain fusing, the zirconia composite sintered body has a surface roughness Ra of preferably 8.0 µm or less after being polished in the polishing step. In view of facilitating reduction of defective porcelain fusing irrespective of the shape of the dental prosthesis even with regions complex in shape and including a number of curved surfaces along with multiple grooves seen in the occlusal aspect, the surface roughness Ra after polishing is more preferably 7.5 µm or less, even more preferably 5.0 µm or less, particularly preferably 2.0 µm or less.

The lower limit of surface roughness Ra is not particularly limited, and may be 0.001 µm or more.

A method for producing a dental prosthesis of the present invention can reduce defective fusing of porcelain by polishing the surface so that the surface roughness Ra falls within the foregoing ranges, in complete contrast to the conventional technique of roughening the surface by sandblasting.

The polishing device and instruments used for polishing are not particularly limited and known polishing devices and instruments can be used, as long as the surface roughness Ra can be adjusted to the foregoing ranges.

In a method for producing a dental prosthesis of the present invention, it is preferable that the zirconia composite sintered body be shaped by machining so that it can be used as a dental prosthesis after building up and firing porcelain.

In a method for producing a dental prosthesis of the present invention, it is preferable that the heat treatment or polishing be performed as a pretreatment immediately before building up porcelain. In other words, in view of facilitating reduction of defective porcelain fusing irrespective of the shape of the dental prosthesis even with regions complex in shape and including a number of curved surfaces along with multiple grooves seen in the occlusal aspect, it is preferable that the heat treatment or polishing is not followed by any further processing for porcelain build-up.

When performed at this point in time, the heat treatment or polishing can reduce defective fusing of porcelain even when the zirconia composite sintered body, serving as the substrate, has initially undergone sandblasting to create surface irregularities. Accordingly, a method for producing a dental prosthesis of the present invention may additionally comprise sandblasting before heat treatment or polishing.

A certain preferred embodiment is, for example, a method for producing a dental prosthesis comprising a pretreatment before (preferably, immediately before) building up porcelain on a zirconia composite sintered body comprising Nb₂O₅ and/or Ta₂O₅, wherein the pretreatment is heat treatment or polishing.

In a method for producing a dental prosthesis of the present invention, porcelain may be built up in any thickness, as long as the present invention can exhibit its effects. For example, when glaze firing is employed, the porcelain may have a thickness of 200 µm or less, 100 µm or less, or 50 µm or less after sintering.

With these porcelain thicknesses, the resulting dental prosthesis can exhibit even superior aesthetics by virtue of the characteristics of porcelain.

The zirconia composite sintered body used in a method for producing a dental prosthesis of the present invention comprises Nb₂O₅ and/or Ta₂O₅.

The processable zirconia described in Patent Literature 2 may be used as the zirconia composite sintered body used in a method for producing a dental prosthesis of the present invention.

Specific examples of the zirconia composite sintered body include a sintered body formed to include a tetragonal composite zirconia comprising 79.8 to 92 mol% ZrO₂, 4.5 to 10.2 mol% Y₂O₃, and either 3.5 to 7.5 mol% Nb₂O₅ or 5.5 to 10.0 mol% Ta₂O₅, together with TiO₂ in a mass percentage of more than 0 mass% and 2.5 mass% or less relative to the composite zirconia.

In a zirconia composite sintered body of the present invention, the zirconia content is preferably 78 to 97.5 mol% in total 100 mol% of zirconia, the stabilizer, Nb₂O₅, and Ta₂O₅. In view of even superior translucency and strength, the zirconia content is more preferably 79 mol% or more and 96 mol% or less, even more preferably 80 mol% or more and 94 mol% or less, particularly preferably 81 mol% or more and 93 mol% or less.

Examples of the stabilizer capable of preventing a phase transformation of zirconia include oxides such as calcium oxide (CaO), magnesium oxide (MgO), yttrium oxide (Y₂O₃), cerium oxide (CeO₂), scandium oxide (Sc₂O₃), lanthanum oxide (La₂O₃), erbium oxide (Er₂O₃), praseodymium oxide (Pr₂O₃, Pr₆O₁₁), samarium oxide (Sm₂O₃), europium oxide (Eu₂O₃), thulium oxide (Tm₂O₃), gallium oxide (Ga₂O₃), indium oxide (In₂O₃), and ytterbium oxide (Yb₂O₃). In view of enhancing the effectiveness of the present invention and providing particularly superior aesthetics, preferred are Y₂O₃ (yttria) and/or CeO₂. The stabilizer may be used alone, or two or more thereof may be used in combination.

In a zirconia composite sintered body of the present invention, the content of the stabilizer is preferably 1 to 12 mol% in total 100 mol% of zirconia, the stabilizer, Nb₂O₅, and Ta₂O₅. In view of ease of achieving sufficient machinability, the stabilizer content is more preferably 2 mol% or more and 10 mol% or less. In view of even superior translucency and strength, the stabilizer content is even more preferably 3 mol% or more and 8.0 mol% or less, yet more preferably 3.5 mol% or more and 7.5 mol% or less, particularly preferably 3.8 mol% or more and 7.0 mol% or less, most preferably 4.0 mol% or more and 6.5 mol% or less.

A certain preferred embodiment is, for example, a zirconia composite sintered body in which the stabilizer capable of preventing a phase transformation of zirconia comprises Y₂O₃ and/or CeO₂, and the combined content of Y₂O₃ and CeO₂ is 2 mol% or more and 10 mol% or less.

Another certain preferred embodiment is, for example, a zirconia composite sintered body in which the stabilizer capable of preventing a phase transformation of zirconia comprises Y₂O₃, and the Y₂O₃ content is 2 mol% or more and 10 mol% or less.

In any of the above embodiments, the content of Y₂O₃ and CeO₂ can be appropriately modified within the ranges indicated in this specification. For example, in view of even superior translucency and strength, the combined content of Y₂O₃ and CeO₂ may be 2.5 mol% or more and 10 mol% or less, or 3 mol% or more and 9 mol% or less.

The reason why a zirconia composite sintered body of the present invention can exhibit superior machinability in the sintered state while maintaining strength is unclear. However, it probably results from enhanced fracture toughness (IF method) and minimized hardness through coarsening of the microstructure, achieved by incorporating at least one of Nb₂O₅ or Ta₂O₅ in addition to conventionally stabilized zirconia.

In a zirconia composite sintered body of the present invention, the content of Nb₂O₅ or Ta₂O₅ is 1 to 9 mol%, preferably 1.5 mol% or more and 8.5 mol% or less in total 100 mol% of zirconia, the stabilizer, Nb₂O₅, and Ta₂O₅. In view of even superior machinability, the content of Nb₂O₅ or Ta₂O₅ is more preferably 2.5 mol% or more and 8 mol% or less, even more preferably 3 mol% or more and 7 mol% or less. When the content of Nb₂O₅ or Ta₂O₅ is less than 1 mol%, it is not easy to achieve sufficient machinability. When the content of Nb₂O₅ or Ta₂O₅ is more than 9 mol%, it tends to cause defects such as chipping in the resulting zirconia composite sintered body, making it difficult to provide sufficient properties.

In view of even superior machinability, it is preferable in certain preferred embodiments that the combined content of Nb₂O₅ and Ta₂O₅ in a zirconia composite sintered body of the present invention fall within the foregoing ranges specified individually for these components (e.g., 1 mol% or more and 9 mol% or less).

In addition to serving to coarsen the microstructure and reduce hardness, and acting together with the capping elements or ions to provide excellent free-machinability as noted above, Nb₂O₅ and Ta₂O₅ also interact with other components (for example, TiO₂, Al₂O₃) incorporated in the zirconia composite sintered body. This interaction, combined with the application of HIP, helps maximize the sintering density, providing natural tooth aesthetics in the resulting dental prosthesis.

The content of zirconia, stabilizer, Nb₂O₅, and Ta₂O₅ represents the proportion of each component in total 100 mol% of zirconia, stabilizer, Nb₂O₅, and Ta₂O₅, and the sum of zirconia, stabilizer, Nb₂O₅, and Ta₂O₅ does not exceed 100 mol%. For example, when the raw material composition contains Nb₂O₅ but does not contain Ta₂O₅, the content of zirconia, stabilizer, and Nb₂O₅ refers to the proportion of each component relative to total 100 mol% of zirconia, stabilizer, and Nb₂O₅.

In view of machinability, the ratio A/B is preferably 0.9 to 3, more preferably 0.95 to 2, where A is the content of stabilizer in mol%, and B is the combined content of Nb₂O₅ and Ta₂O₅ in mol%. Even more preferably, the ratio A/B is 1 to 1.6 in view of enhancing the effectiveness of the combined action between the capping elements or ions and Nb₂O₅ and/or Ta₂O₅ and improving free-machinability, and increasing the number of dental prostheses that can be produced in continuous processing with a single processing tool while reducing wear on processing tools.

A certain preferred embodiment is, for example, a method for producing a dental prosthesis in which the zirconia composite sintered body comprises zirconia, a stabilizer capable of preventing a phase transformation of zirconia (hereinafter, also referred to simply as "stabilizer"), and Nb₂O₅ and/or Ta₂O₅, wherein the zirconia content is 78 to 97.5 mol%, the stabilizer content is 1 to 12 mol%, and the content of Nb₂O₅ and/or Ta₂O₅ is 1 to 9 mol% in total 100 mol% of zirconia, the stabilizer, Nb₂O₅, and Ta₂O₅.

In the production method of a dental prosthesis of such certain preferred embodiment, an element or ion derived form a capping agent (described later) may be absent.

Another certain preferred embodiment is, for example, a method for producing a dental prosthesis in which the zirconia composite sintered body comprises zirconia, a stabilizer, and Nb₂O₅ and/or Ta₂O₅, wherein the zirconia content is 78 to 97.5 mol%, the stabilizer content is 1 to 12 mol%, and the content of Nb₂O₅ and/or Ta₂O₅ is 1 to 9 mol% in total 100 mol% of zirconia, the stabilizer, Nb₂O₅, and Ta₂O₅, with an element or ion derived form a capping agent additionally incorporated in the zirconia composite sintered body.

In this specification, elements or ions derived from a capping agent (hereinafter, also referred to as "capping elements or ions") refer to elements or ions that weaken the strength of the crystal interface (hereinafter, also referred to as "grain boundary") by capping the terminals of the bonding sites of zirconia-based composite oxides within a zirconia composite sintered body constituted of zirconia-based composite oxides including Nb₂O₅ and/or Ta₂O₅ (hereinafter, the strength of grain boundaries is also referred to as "grain boundary strength").

The capping agent can partially cap the crystal grain boundaries.

The term "capping" refers to elements or ions (capping elements or ions) of interest existing at the crystal grain boundaries by binding to the bonding sites of zirconia-based composite oxides instead of the metal elements.

It is believed that the presence of capping elements or ions in the form of +1 cations or -1 anions at the grain boundaries causes electrostatic repulsion between these capping cations or anions, thereby reducing the grain boundary strength.

The content of zirconia, stabilizer, Nb₂O₅ and Ta₂O₅ in the zirconia composite sintered body can be individually measured using a technique, for example, such as inductively coupled plasma (ICP) emission spectral analysis or X-ray fluorescence analysis.

The content (mol%) of elements or ions derived from a capping agent refers to the proportion external to total 100 mol% of zirconia, the stabilizer, Nb₂O₅, and Ta₂O₅. Accordingly, the content of the elements or ions derived from a capping agent in the zirconia composite sintered body can be calculated by converting the quantity (mass) of the raw material added into mol%.

In the zirconia composite sintered body comprising capping elements or ions, the capping elements or ions present at the crystal grain boundaries appear to reduce the grain boundary strength in the form of +1 cations or -1 anions, facilitating particles to separate, thereby improving grindability and machinability in the sintered state.

In the form of +1 cations or -1 anions, the capping elements or ions bind to the bonding sites of zirconia-based composite oxides at the grain boundaries of the zirconia composite sintered body. This binding leads to electrostatic repulsion between the cations or anions, reducing the grain boundary strength while maintaining the strength and translucency properties of the particles constituting the zirconia composite sintered body, resulting in improved machinability in the sintered state.

As a possible example of such bonding, a +1 cation can form a bond with the other bonding site of an oxygen atom that is already bonded to the metal element (for example, Zr, Hf, Y, Nb, or Ta) contained in the zirconia-based composite oxide, instead of these metal elements forming such bonds.

Another possible example is where a -1 anion binds to the OH₂⁺ attached to the metal element (for example, Zr, Hf, Y, Nb, or Ta) contained in the zirconia-based composite oxide.

In yet another possible example, a -1 anion can bind to the cation derived from the metal element (for example, Zr, Hf, Y, Nb, or Ta) contained within the zirconia-based composite oxide and attached to other metal elements.

In the zirconia composite sintered body, Nb₂O₅ and/or Ta₂O₅ serve to coarsen the microstructure and reduce hardness, and, by acting together with the capping elements or ions, improve machinability. Through this combined action, Nb₂O₅ and/or Ta₂O₅ and the capping elements or ions can provide even superior free-machinability while ensuring the strength needed as artificial teeth, reducing machining time and increasing the number of dental prostheses that can be produced in continuous processing with a single processing tool while reducing wear on processing tools.

In a zirconia composite sintered body of the present invention, the capping elements or ions further improve machinability, and serve as a free-cutting aid in the manner described above, without greatly compromising strength and translucency.

The content of the capping elements or ions contained in the zirconia composite sintered body used in the present invention is preferably more than 0 mol% and 5 mol% or less. In view of providing even superior machinability and increasing the number of dental prostheses that can be produced in continuous processing with a single processing tool, the content of capping elements or ions is more preferably 0.05 mol% or more and 3 mol% or less, even more preferably 0.06 mol% or more and 2.5 mol% or less, particularly preferably 0.07 mol% or more and 1.0 mol% or less, most preferably 0.08 mol% or more and 0.34 mol% or less.

When the capping elements or ions contained in the zirconia composite sintered body used in the present invention are group 17 elements or ions, the content of capping elements or ions is more preferably 0.2 mol% or more and 5 mol% or less, even more preferably 0.3 mol% or more and 4 mol% or less, particularly preferably 0.4 mol% or more and 3.5 mol% or less, most preferably 0.5 mol% or more and 3.0 mol% or less in view of providing even superior machinability and increasing the number of dental prostheses that can be produced in continuous processing with a single processing tool.

As described above, the capping elements or ions at the grain boundaries exist in the form of +1 cations or -1 anions, ensuring appropriate interactions between charged sites and adsorption sites at the grain boundaries.

For capping elements or ions, it is preferable to use elements or ions thereof that belong to periods 2 to 7 of the periodic table, and are elements or ions thereof having a lower first ionization energy than the elements in group 18 of the same periods, or elements or ions thereof with high electron affinity. Also preferred are nitrate ions, hypochlorite ions, chlorite ions, chlorate ions, perchlorate ions, bromate ions, permanganate ions, metaborate ions, and cyanide ions.

A certain preferred embodiment is, for example, a zirconia composite sintered body in which the elements or ions thereof derived from a capping agent belong to periods 2 to 7 of the periodic table, and are elements or ions thereof having a lower first ionization energy than the elements in group 18 of the same periods, and/or elements or ions thereof with high electron affinity.

In view of facilitating the formation of +1 cations and providing even superior machinability, the elements belonging to periods 2 to 7 of the periodic table and having a lower first ionization energy than the elements in group 18 of the same periods are preferably Cu, Ag, Li, Na, K, Rb, Cs, and Fr, for example.

In view of facilitating the formation of -1 anions and providing even superior machinability, the elements with high electron affinity are preferably group 17 elements. Preferred as group 17 elements are At, I, Br, Cl, and F.

The first ionization energy refers to the energy needed to remove one electron from a neutral atom to ionize it. The first ionization energy can be referenced from Shriver & Atkins Inorganic Chemistry (Volume 1), 4th Edition, Part 1: Foundations, 1. Atomic Structure. The first ionization energy, given in eV in Appendix 2 of this book, can be converted into KJ/mol using the conversion rate of 1eV = 96.485 KJ/mol. The first ionization energy can also be determined using photoelectron yield spectroscopy (PYS).

Electron affinity (EA) is the amount of energy released when an electron is incorporated in a neutral atom. Electron affinity can be determined by the difference between the ionization potential and the energy gap. The ionization potential is defined as the energy difference between the highest occupied molecular orbital of a compound and the vacuum level, and it is measured using ultraviolet photoelectron spectroscopy.

For the first ionization energy and electron affinity, data available in the NIST Chemistry WebBook (https://webbook.nist.gov/chemistry/) may be utilized (by selecting either Ionization Energy or Electron Affinity from Ion energetics properties).

The methods described above can be appropriately employed for the measurement of first ionization energy and electron affinity because the measurement simply involves comparing the likelihood to form +1 cations or -1 anions among different elements.

Specific examples of the capping elements include Cu, Ag, Li, Na, K, Rb, Cs, Fr, At, I, Br, Cl, and F. In view of the ability to more greatly improve machinability, preferred are Cu, Ag, Li, Na, K, Rb, Cs, Fr, I, Br, Cl, and F, more preferably Li, Na, K, Rb, Cs, and Fr.

A certain preferred embodiment is, for example, a zirconia composite sintered body in which the element derived from a capping agent comprises at least one element selected from the group consisting of Cu, Ag, Li, Na, K, Rb, Cs, Fr, At, I, Br, Cl, and F, and ions of these elements are at least one +1 cation or -1 anion selected from the group consisting of Cu, Ag, Li, Na, K, Rb, Cs, Fr, I, Br, Cl, and F.

Another preferred embodiment is, for example, a zirconia composite sintered body in which the element or ion derived from a capping agent comprises at least one element or ion thereof selected from the group consisting of Ag, Li, Na, K, Rb, Cs, Fr, At, I, Br, Cl, and F.

The capping element or ion may be used alone, or two or more thereof may be used in combination.

As described above, the capping elements or ions act together with Nb₂O₅ and/or Ta₂O₅, and these do not compromise the effectiveness of the stabilizer. Accordingly, the present invention can exhibit its effects without particularly restricting the choice of stabilizer.

In the zirconia composite sintered body comprising a capping element or ion, the type of stabilizer, and the content of zirconia and stabilizer may be the as in the embodiments described above.

In the zirconia composite sintered body comprising a capping element or ion, the content of Nb₂O₅ or Ta₂O₅ is 1 to 9 mol%, preferably 1.5 mol% or more and 8.5 mol% or less in total 100 mol% of zirconia, the stabilizer, Nb₂O₅, and Ta₂O₅. In view of providing even superior machinability through combined action with the capping element or ion, the content of Nb₂O₅ or Ta₂O₅ is more preferably 2.5 mol% or more and 8 mol% or less, even more preferably 3 mol% or more and 7 mol% or less. When the content of Nb₂O₅ or Ta₂O₅ is less than 1 mol%, it is not easy to achieve sufficient machinability. When the content of Nb₂O₅ or Ta₂O₅ is more than 9 mol%, it tends to cause defects such as chipping in the resulting zirconia composite sintered body, making it difficult to provide sufficient properties.

In view of even superior machinability, it is preferable in certain preferred embodiments that the combined content of Nb₂O₅ and Ta₂O₅ in the zirconia composite sintered body comprising a capping element or ion fall within the foregoing ranges specified individually for these components (e.g., 1 mol% or more and 9 mol% or less).

A certain preferred embodiment of the present invention is, for example, a zirconia composite sintered body that comprises zirconia, a stabilizer capable of preventing a phase transformation of zirconia, and Nb₂O₅ and/or Ta₂O₅, and in which the zirconia content is 78 to 97.5 mol%, the stabilizer content is 1 to 12 mol%, and the content of Nb₂O₅ and/or Ta₂O₅ is 1 to 9 mol% in total 100 mol% of zirconia, the stabilizer, Nb₂O₅, and Ta₂O₅,
wherein the zirconia composite sintered body additionally comprises a capping element or ion,
the stabilizer comprises Y₂O₃ and/or CeO₂,
the content of the capping element or ion is more than 0 mol% and 5 mol% or less relative to total 100 mol% of zirconia, the stabilizer, Nb₂O₅, and Ta₂O₅, and
the zirconia composite sintered body has a ratio A/B of 0.9 to 3, where A is the content of the stabilizer in mol%, and B is the combined content of Nb₂O₅ and Ta₂O₅ in mol%.

Another example of the zirconia composite sintered body used in the present invention is a zirconia composite sintered body that comprises a zirconia enhancer, in addition to zirconia, a stabilizer capable of preventing a phase transformation of zirconia, Nb₂O₅ and/or Ta₂O₅, and a capping element or ion.

In the zirconia composite sintered body comprising zirconia, a stabilizer capable of preventing a phase transformation of zirconia, and Nb₂O₅ and/or Ta₂O₅, the zirconia enhancer can improve the strength of the sintered body through combined action with the capping elements or ions.

In the zirconia composite sintered body comprising a zirconia enhancer, it is also possible to appropriately modify the zirconia content, the type and content of stabilizer, the content of Nb₂O₅ and/or Ta₂O₅, the type and content of capping elements or ions, and the ratio A/B, as noted above.

In the zirconia composite sintered body comprising a zirconia enhancer, the content of the zirconia enhancer is preferably more than 0 mass% and 6.0 mass% or less relative to total 100 mass% of zirconia, the stabilizer capable of preventing a phase transformation of zirconia, Nb₂O₅, and Ta₂O₅. In view of achieving even greater strength together with capping elements or ions when combined with capping elements or ions, the content of the zirconia enhancer is more preferably 0.01 mass% or more and 5.5 mass% or less, even more preferably 0.5 mass% or more and 5.0 mass% or less.

Examples of the zirconia enhancer include TiO₂ and Al₂O₃. The zirconia enhancer may be used alone, or two or more thereof may be used in combination.

Another example of the zirconia composite sintered body used in the present invention is a zirconia composite sintered body in which the zirconia enhancer comprises TiO₂, and the content of TiO₂ is 0.6 to 4.5 mass%.

Yet another example of the zirconia composite sintered body used in the present invention is a zirconia composite sintered body that comprises zirconia, a stabilizer capable of preventing a phase transformation of zirconia, and Nb₂O₅ and/or Ta₂O₅, and in which the zirconia content is 78 to 97.5 mol%, the stabilizer content is 1 to 12 mol%, and the content of Nb₂O₅ and/or Ta₂O₅ is 1 to 9 mol% in total 100 mol% of zirconia, the stabilizer, Nb₂O₅, and Ta₂O₅,
wherein the zirconia composite sintered body additionally comprises a capping element or ion,
the stabilizer comprises Y₂O₃ and/or CeO₂,
the zirconia enhancer comprises TiO₂, and the content of TiO₂ is 0.6 to 4.5 mass%,
the content of the capping element or ion is more than 0 mol% and 5 mol% or less relative to total 100 mol% of zirconia, the stabilizer, Nb₂O₅, and Ta₂O₅, and
the zirconia composite sintered body has a ratio A/B of 0.9 to 3, where A is the content of the stabilizer in mol%, and B is the combined content of Nb₂O₅ and Ta₂O₅ in mol%.

The zirconia composite sintered body used in the present invention has an average crystal grain size of preferably 0.5 to 5.0 µm. In view of even superior machinability, strength, and translucency, the average crystal grain size is more preferably 0.5 to 4.5 µm, even more preferably 0.7 to 4.0 µm. As an example, the following method can be used to measure the average crystal grain size.

First, a surface image of a pellet-shaped zirconia composite sintered body is captured using a scanning electron microscope (VE-9800 manufactured by Keyence under this trade name). The average crystal grain size is then calculated by image analysis after indicating grain boundaries on individual crystal grains in the image.

For the measurement of average crystal grain size, the captured SEM image is binarized with image analysis software (Image-Pro Plus manufactured by Hakuto Co., Ltd. under this trade name), and particles are recognized from the field (region) by adjusting the brightness range to provide clear grain boundaries. The crystal grain size from Image-Pro Plus is the average of the measurements of the length of a line segment connecting the contour line and passing through the centroid determined from the contour line of the crystal grain, conducted at 2-degree intervals with the centroid as the central point. The measurement of crystal grain size is conducted for all particles not lying on the edges of the SEM photographic image (3 fields) of each Example and Comparative Example. The arithmetic average of the measured crystal grain sizes is then determined as the average crystal grain size (number-based) of the sintered body.

Note that "particles not lying on the edges of the image" refers to particles excluding those with contour lines extending beyond the frame of the SEM photographic image (particles with their contour lines interrupted by the boundary lines at the top, bottom, left, and right). To select the crystal grain size of all particles not lying on the edges of the image, the option in Image-Pro Plus can be used that excludes all particles lying on the boundary lines.

The average crystal grain size can be measured by adjusting the particle count in one SEM image field to about 50 or more, 100 or more, 200 or more, or 600 or more (for example, about 800 or 1,000 particles), according to the method described in the EXAMPLES below.

In a zirconia composite sintered body according to the present invention, higher densities result in fewer internal voids, improved translucency due to less light scattering, and enhanced strength. In view of this, the density of the zirconia composite sintered body is preferably 5.5 g/cm³ or more, more preferably 5.7 g/cm³ or more, even more preferably 5.9 g/cm³ or more.

Particularly preferably, the zirconia composite sintered body is essentially void free.

The density of the composite sintered body can be calculated by dividing its mass by its volume ((mass of composite sintered body) / (volume of composite sintered body)).

As indicated above, the processable zirconia described in Patent Literature 2 may be used as the zirconia composite sintered body used in the present invention. The processable zirconia can be produced according to the production method described in Patent Literature 2.

The following describes a method for producing a zirconia composite sintered body, based on the case where the zirconia composite sintered body used in a method for producing a dental prosthesis of the present invention comprises a capping element or ion.

An example of a method for producing a zirconia composite sintered body comprising a capping element or ion comprises the steps of:
fabricating a molded body using a raw material composition that comprises zirconia, a stabilizer capable of preventing a phase transformation of zirconia, and Nb₂O₅ and/or Ta₂O₅, and in which the zirconia content is 78 to 97.5 mol%, the stabilizer content is 1 to 12 mol%, and the content of Nb₂O₅ and/or Ta₂O₅ is 1 to 9 mol% in total 100 mol% of zirconia, the stabilizer, Nb₂O₅, and Ta₂O₅, with a capping agent additionally incorporated in the raw material composition; and
sintering the molded body.

The raw material composition of the zirconia composite sintered body comprises zirconia, a stabilizer capable of preventing a phase transformation of zirconia, Nb₂O₅ and/or Ta₂O₅, and a capping agent. The raw material composition of the zirconia composite sintered body may be in a dry state, or in a liquid state containing liquid or contained in liquid. For example, the raw material composition may have a form of a powder, a granule or granulated material, a paste, or a slurry.

The raw material composition comprises a capping agent so that the zirconia composite sintered body produced comprises a capping element or ion.

The capping agent is not particularly limited, as long as it is a compound with the potential to become a monovalent ion (+1 cation or -1 anion) in a solvent containing water. Examples include hydroxides, salts, halides (fluorides, chlorides, bromides, iodides), and cyanides containing elements or ions derived from a capping agent. The capping agent may be used alone, or two or more thereof may be used in combination.

Examples of hydroxides containing capping elements or ions include lithium hydroxide, sodium hydroxide, potassium hydroxide, rubidium hydroxide, cesium hydroxide, and francium hydroxide.

Examples of salts containing capping elements or ions include carbonates, bicarbonates, nitrates, hypochlorites, chlorites, chlorates, perchlorates, bromates, permanganates, metaborates, sulfides, and cyanides.

Examples of carbonates containing capping elements or ions include lithium carbonate, sodium carbonate, potassium carbonate, rubidium carbonate, francium carbonate, and cesium carbonate.

Examples of bicarbonates containing capping elements or ions include lithium bicarbonate, sodium bicarbonate, potassium bicarbonate, rubidium bicarbonate, francium bicarbonate, and cesium bicarbonate.

Examples of nitrates containing capping elements or ions include calcium nitrate, strontium nitrate, iron(II) nitrate, iron(III) nitrate, cobalt(II) nitrate, magnesium nitrate, gallium nitrate, yttrium(III) nitrate, lanthanum(III) nitrate, praseodymium nitrate, neodymium(III) nitrate, manganese(II) nitrate, europium nitrate, copper(II) nitrate, thorium nitrate, aluminum nitrate, nickel(II) nitrate, chromium(III) nitrate, titanium(IV) nitrate, zirconium nitrate, zirconium(IV) oxynitrate hydrate (ZrO(NO₃)₂·xH₂O), cerium(III) nitrate, tin nitrate, bismuth(III) nitrate, scandium(III) nitrate, indium(III) nitrate, and hafnium(IV) nitrate.

Examples of hypochlorites containing capping elements or ions include sodium hypochlorite, and calcium hypochlorite.

Examples of chlorites containing capping elements or ions include sodium chlorite, potassium chlorite, lithium chlorite, calcium chlorite, magnesium chlorite, barium chlorite, copper(II) chlorite, copper(III) chlorite, silver chlorite, and nickel chlorite.

Examples of chlorates containing capping elements or ions include calcium chlorate, barium chlorate, cobalt chlorate, nickel chlorate, magnesium chlorate, zinc chlorate, and copper chlorate.

Examples of perchlorates containing capping elements or ions include iron(III) perchlorate, barium perchlorate, calcium perchlorate, cobalt perchlorate, nickel perchlorate, magnesium perchlorate, beryllium perchlorate, aluminum perchlorate, and cerium perchlorate.

Examples of bromates containing capping elements or ions include neodymium bromate, lanthanum bromate, and praseodymium bromate.

Examples of permanganates containing capping elements or ions include calcium(VII) permanganate, potassium(VII) permanganate, and sodium(VII) permanganate.

Examples of metaborates containing capping elements or ions include sodium metaborate, and barium metaborate.

Examples of sulfides containing capping elements or ions include copper(I) sulfide.

Examples of cyanides containing capping elements or ions include barium cyanide, sodium cyanide, potassium cyanide, and calcium cyanide.

Examples of fluorides containing capping elements or ions include lithium fluoride, sodium fluoride, potassium fluoride, rubidium fluoride, cesium fluoride, francium fluoride, beryllium fluoride, magnesium fluoride, calcium fluoride, strontium fluoride, barium fluoride, scandium(III) fluoride, yttrium(III) fluoride, lanthanum(III) fluoride, cerium(III) fluoride, neodymium(III) fluoride, titanium(III) fluoride, titanium(IV) fluoride, zirconium(IV) fluoride, hafnium(IV) fluoride, tantalum(V) fluoride, manganese(II) fluoride, manganese(III) fluoride, iron(II) fluoride, iron(III) fluoride, copper(II) fluoride, zinc(II) fluoride, aluminum fluoride, chromium(III) fluoride, bismuth(III) fluoride, indium(III) fluoride, and tin(II) fluoride.

Examples of chlorides containing capping elements or ions include zirconium oxychloride, lithium chloride, sodium chloride, potassium chloride, rubidium chloride, cesium chloride, francium chloride, beryllium chloride, magnesium chloride, calcium chloride, strontium chloride, barium chloride, scandium(III) chloride, yttrium(III) chloride, lanthanum(III) chloride, cerium(III) chloride, praseodymium chloride, neodymium(III) chloride, samarium chloride, europium chloride, titanium(III) chloride, titanium(IV) chloride, zirconium(IV) chloride, hafnium(IV) chloride, tantalum(V) chloride, manganese chloride, iron(II) chloride, iron(III) chloride, cobalt(II) chloride, nickel(II) chloride, copper(I) chloride, copper(II) chloride, zinc(II) chloride, aluminum chloride, gallium chloride, bismuth(III) chloride, indium(I) chloride, indium(III) chloride, and tin(II) chloride.

Examples of bromides containing capping elements or ions include lithium bromide, sodium bromide, potassium bromide, rubidium bromide, cesium bromide, francium bromide, beryllium bromide, magnesium bromide, calcium bromide, strontium bromide, barium bromide, scandium(III) bromide, yttrium(III) bromide, cerium(III) bromide, neodymium(III) bromide, titanium(IV) bromide, zirconium(IV) bromide, tantalum(V) bromide, manganese(II) bromide, iron(II) bromide, iron(III) bromide, cobalt(II) bromide, nickel(II) bromide, copper(I) bromide, copper(II) bromide, zinc(II) bromide, chromium(III) bromide, bismuth(III) bromide, vanadium(III) bromide, indium(III) bromide, and tin bromide.

Examples of iodides containing capping elements or ions include iodides such as lithium iodide, sodium iodide, potassium iodide, rubidium iodide, cesium iodide, francium iodide, beryllium iodide, calcium iodide, magnesium iodide, strontium iodide, barium iodide, scandium(III) iodide, yttrium(III) iodide, lanthanum(III) iodide, cerium(III) iodide, neodymium(III) iodide, titanium(IV) iodide, zirconium(IV) iodide, hafnium(IV) iodide, tantalum(V) iodide, manganese(II) iodide, iron(II) iodide, iron(III) iodide, cobalt(II) iodide, nickel(II) iodide, copper(I) iodide, zinc(II) iodide, aluminum iodide, chromium(III) iodide, vanadium(II) iodide, bismuth(III) iodide, indium(III) iodide, tin(II) iodide, and tin(IV) iodide.

Commercially available zirconia powders can be used for zirconia.

Examples of such commercially available products include zirconia powders manufactured by Tosoh Corporation under the trade names Zpex^{®} (Y₂O₃ content: 3 mol%), Zpex^{®} 4 (Y₂O₃ content: 4 mol%), Zpex^{®} Smile^{®} (Y₂O₃ content: 5.5 mol%), TZ-3Y (Y₂O₃ content: 3 mol%), TZ-3YS (Y₂O₃ content: 3 mol%), TZ-4YS (Y₂O₃ content: 4 mol%), TZ-6Y (Y₂O₃ content: 6 mol%), TZ-6YS (Y₂O₃ content: 6 mol%), TZ-8YS (Y₂O₃ content: 8 mol%), TZ-10YS (Y₂O₃ content: 10 mol%), TZ-3Y-E (Y₂O₃ content: 3 mol%), TZ-3YS-E (Y₂O₃ content: 3 mol%), TZ-3YB-E (Y₂O₃ content: 3 mol%), TZ-3YSB-E (Y₂O₃ content: 3 mol%), TZ-3YB (Y₂O₃ content: 3 mol%), TZ-3YSB (Y₂O₃ content: 3 mol%), TZ-3Y20AB (Y₂O₃ content: 3 mol%), TZ-8YSB (Y₂O₃ content: 8 mol%), and TZ-0 (Y₂O₃ content: 0 mol%). These commercially available zirconia powders also contain HfO₂. It is also possible to use commercially available products additionally containing Y₂O₃.

The raw material composition of the present invention may also use zirconia powders in which Y₂O₃ is uniformly dispersed as a solid solution, as in the TZ series of the commercially available products listed above (those with "TZ" in the product names).

The method of production of zirconia powder is not particularly limited, and known methods may be employed, for example, such as the breakdown process, where coarse particles are pulverized into fine powder, or the building-up process, where synthesis occurs through nucleation and growth from atoms or ions.

The type of zirconia powder in the raw material composition is not particularly limited. It is possible to additionally incorporate stabilizer particles when the zirconia powder comprises zirconia but does not contain a stabilizer, or when increasing the content of stabilizer as needed. The stabilizer particles are not particularly limited, as long as the content of the stabilizer within the zirconia composite sintered body can be adjusted to the predetermined ranges mentioned above.

For example, the stabilizer particles may be commercially available products, and may be prepared by pulverizing a commercially available powder using a known pulverizing mixer (such as a ball mill).

The stabilizer may be either a stabilizer not dissolved in zirconia as a solid solution, or a stabilizer dissolved in zirconia as a solid solution.

In view of ease of obtaining the desired zirconia composite sintered body, a certain preferred embodiment is, for example, a method for producing a zirconia composite sintered body in which the stabilizer (preferably, Y₂O₃) in the raw material composition comprises a stabilizer not dissolved in zirconia as a solid solution. The presence of stabilizers not dissolved in zirconia as a solid solution can be verified, for example, through X-ray diffraction (XRD) patterns.

The presence of peaks derived from the stabilizer in an XRD pattern of the raw material composition or molded body indicates the presence of a stabilizer that is not dissolved in zirconia in the raw material composition or molded body.

A peak derived from the stabilizer is basically not observable in an XRD pattern when the stabilizer is fully dissolved in zirconia as a solid solution. It is, however, possible, depending on the crystal state or other conditions of the stabilizer, that the stabilizer is not dissolved in zirconia as a solid solution even when the XRD pattern does not show peaks for stabilizers.

The following describes situations where the stabilizer comprises a stabilizer not dissolved in zirconia as a solid solution, with yttria serving as an example of the stabilizer.

In the raw material composition or molded body of the present invention, the abundance f_{y} of yttria not dissolved in zirconia as a solid solution (hereinafter, also referred to as "undissolved yttria") can be calculated using the following mathematical formula (1). ${f}_{y} = {I}_{29} / \left({I}_{28}+{I}_{29}+{I}_{30}\right) \times 100$ (1) where f_{y} represents the fraction (%) of undissolved yttria, I₂₈ represents the integrated intensity of a peak near 2θ = 28°, where the main peak of monoclinic zirconia appears in XRD measurement, I₂₉ represents the integrated intensity of a peak near 2θ = 29°, where the main peak of yttria appears in XRD measurement, and I₃₀ represents the integrated intensity of a peak near 2θ = 30°, where the main peak of tetragonal or cubic zirconia appears in XRD measurement.

When additionally using stabilizers other than yttria, the formula can be applied to calculate the abundance of undissolved stabilizers other than yttria by substituting I₂₉ with the peaks of other stabilizers.

In view of considerations such as ease of obtaining the desired zirconia composite sintered body, the abundance f_{y} of undissolved yttria is preferably higher than 0%, more preferably 1% or more, even more preferably 2% or more, particularly preferably 3% or more. The upper limit for the abundance f_{y} of undissolved yttria may be, for example, 25% or less. Preferably, the upper limit of abundance f_{y} of undissolved yttria depends on the yttria content in the raw material composition or molded body.

For example, the abundance f_{y} of undissolved yttria falls within the following ranges when the yttria content in the raw material composition or molded body of the present invention is 3 mol% or more and 8 mol% or less.

When the yttria content is 3 mol% or more and less than 4.5 mol%, f_{y} may be 15% or less. When the yttria content is 4.5 mol% or more and less than 5.8 mol%, f_{y} may be 20% or less. When the yttria content is 5.8 mol% or more and 8 mol% or less, f_{y} may be 25% or less.

For example, when the yttria content is 3 mol% or more and less than 4.5 mol%, f_{y} is preferably 2% or more, more preferably 3% or more, even more preferably 4% or more, particularly preferably 5% or more.

When the yttria content is 4.5 mol% or more and less than 5.8 mol%, f_{y} is preferably 3% or more, more preferably 4% or more, even more preferably 5% or more, yet more preferably 6% or more, particularly preferably 7% or more.

When the yttria content is 5.8 mol% or more and 8 mol% or less, f_{y} is preferably 4% or more, more preferably 5% or more, even more preferably 6% or more, yet more preferably 7% or more, particularly preferably 8% or more.

In the raw material composition or molded body of the present invention, it is not necessarily required for the stabilizer to be fully dissolved in zirconia as a solid solution. In the present invention, "stabilizer being dissolved as a solid solution" means that, for example, the elements (atoms) contained in the stabilizer are dissolved in zirconia as a solid solution.

There is no particular restriction on Nb₂O₅ and/or Ta₂O₅ incorporated in the raw material composition of the present invention, as long as the content of Nb₂O₅ and/or Ta₂O₅ within the zirconia composite sintered body can be adjusted in the predetermined ranges mentioned above. For example, commercially available products may be used for Nb₂O₅ and/or Ta₂O₅ without any particular restrictions. These may be used after pulverizing its powder using a known pulverizing mixer (such as a ball mill).

An example method of obtaining the raw material composition involves a process whereby the raw material composition is prepared by, for example, wet mixing each raw material of the raw material composition (zirconia, a stabilizer, and optionally, components such as at least one of Nb₂O₅ or Ta₂O₅, a capping agent (for example, a compound with the potential to become a monovalent ion in a solvent containing water), and a zirconia enhancer) in a solvent containing water.

The method for wet mixing the raw materials in a solvent containing water is not particularly limited. For example, the raw materials may be formed into a slurry through wet pulverization and mixing using a known pulverizing mixer (such as a ball mill). The slurry can then be dried and granulated to produce a granular raw material composition.

In the wet mixing process, it is possible to additionally include additives such as binders, plasticizers, dispersants, emulsifiers, antifoaming agents, pH adjusters, and lubricants. The additives may be used alone, or two or more thereof may be used in combination.

The binder may be added to the pulverized slurry after the slurry is formed by adding a primary powder mixture of zirconia, Y₂O₃, Nb₂O₅ and/or Ta₂O₅, and a capping agent into water.

The binder is not particularly limited, and known binders may be used. Examples of the binders include polyvinyl alcohol binders, acrylic binders, wax binders (such as paraffin wax), methyl cellulose, carboxymethyl cellulose, polyvinyl butyral, polymethylmethacrylate, ethyl cellulose, polyethylene, polypropylene, ethylene-vinyl acetate copolymer, polystyrene, atactic polypropylene, and methacrylic resin.

Examples of the plasticizers include polyethylene glycol, glycerin, propylene glycol, and dibutyl phthalic acid.

Examples of the dispersants include ammonium polycarboxylates (such as triammonium citrate), ammonium polyacrylate, acrylic copolymer resin, acrylic acid ester copolymer, polyacrylic acid, bentonite, carboxymethyl cellulose, anionic surfactants (for example, polyoxyethylene alkyl ether phosphoric acid esters such as polyoxyethylene lauryl ether phosphoric acid ester), non-ionic surfactants, oleic glyceride, amine salt surfactants, oligosaccharide alcohols, and stearic acid.

Examples of the emulsifiers include alkyl ethers, phenyl ethers, and sorbitan derivatives.

Examples of the antifoaming agents include alcohols, polyethers, silicone, and waxes.

Examples of the pH adjusters include ammonia, and ammonium salts (including ammonium hydroxides such as tetramethylammonium hydroxide).

Examples of the lubricants include polyoxyethylene alkyl ethers, and waxes.

The solvent used for wet mixing is not particularly limited, as long as it contains water. By using organic solvents, the solvent may be a mixed solvent of water and organic solvent. Alternatively, the solvent may be solely water. Examples of the organic solvents include ketone solvents such as acetone, and ethyl methyl ketone; and alcohol solvents such as ethanol, 1-propanol, 2-propanol, 2-methyl-2-propanol, glycerin, diglycerin, polyglycerin, propylene glycol, dipropylene glycol, polypropylene glycol, ethylene glycol, diethylene glycol, polyethylene glycol, polyethylene glycol monomethyl ether, 1,2-pentadiol, 1,2-hexanediol, and 1,2-octanediol.

The raw material composition of the zirconia composite sintered body used in the present invention may optionally comprise other additional components aside from a zirconia enhancer, in addition to ZrO₂, Y₂O₃, Nb₂O₅, Ta₂O₅, and a capping agent, provided that the present invention can exhibit its effects. Examples of such additional components include colorants (pigments and composite pigments), fluorescent agents, and SiO₂. The additional components may be used alone, or two or more thereof may be used in combination.

Examples of the pigments include oxides of at least one element selected from the group consisting of V, Cr, Mn, Fe, Co, Ni, Zn, Y, Zr, Sn, Sb, Bi, Ce, Pr, Sm, Eu, Gd, Tb, and Er (specifically, such as NiO and Cr₂O₃), preferably oxides of at least one element selected from the group consisting of V, Cr, Mn, Fe, Co, Ni, Zn, Y, Zr, Sn, Sb, Bi, Ce, Pr, Sm, Eu, Gd, and Tb, more preferably oxides of at least one element selected from the group consisting of V, Cr, Mn, Fe, Co, Ni, Zn, Y, Zr, Sn, Sb, Bi, Ce, Sm, Eu, Gd, and Tb. The pigments may exclude Y₂O₃ and CeO₂.

Examples of the composite pigments include (Zr,V)O₂, Fe(Fe,Cr)₂O₄, (Ni,Co,Fe)(Fe,Cr)₂O₄·ZrSiO₄, and (Co,Zn)Al₂O₄.

Examples of the fluorescent agents include Y₂SiO₅:Ce, Y₂SiO₅:Tb, (Y,Gd,Eu)BO₃, Y₂O₃:Eu, YAG:Ce, ZnGa₂O₄:Zn, and BaMgAl₁₀O₁₇:Eu.

Following its production, the raw material composition is molded to fabricate a molded body. The molding method is not particularly limited, and known molding methods can be used (for example, such as press molding).

When producing a zirconia molded body using a method that includes press molding the raw material composition, there are no specific limitations on the press molding method, and press molding may be achieved using known press molding machines. Specific examples of press molding methods include uniaxial pressing.

The molding pressure is appropriately optimized according to the desired size of molded body, open porosity, biaxial flexural strength, and the particle size of raw material powder. Typically, the pressure in press molding ranges from 5 MPa to 1,000 MPa. By increasing the molding pressure in the method of production above, it is possible to achieve more effective closure of pores in the resulting molded body, leading to a lower open porosity and increasing the density of the molded body. To increase the density of the zirconia molded body obtained, cold isostatic pressing (CIP) may be applied after uniaxial pressing.

Following its production, the molded body is sintered to obtain a zirconia composite sintered body.

The sintering temperature (highest sintering temperature) for sintering the molded body to obtain the zirconia composite sintered body is, for example, preferably 1,300°C or more, more preferably 1,350°C or more, even more preferably 1,400°C or more, yet more preferably 1,450°C or more, particularly preferably 1,500°C or more. The sintering temperature is, for example, preferably 1,680°C or less, more preferably 1,650°C or less, even more preferably 1,600°C or less. Preferably, the method for producing the zirconia composite sintered body used in the present invention comprises the step of sintering the molded body at a highest sintering temperature of 1,300 to 1,680°C. The highest sintering temperature is preferably a temperature in the atmosphere.

The hold time (retention time) at the highest sintering temperature is preferably 30 hours or less, more preferably 20 hours or less, even more preferably 10 hours or less, yet more preferably 5 hours or less, particularly preferably 3 hours or less, most preferably 2 hours or less, depending on the temperature. The hold time may be 25 minutes or less, 20 minutes or less, or 15 minutes or less. The hold time is preferably 1 minute or more, more preferably 5 minutes or more, even more preferably 10 minutes or more. A production method of the present invention enables the fabrication of a zirconia composite sintered body having superior flexural strength, translucency, and machinability in a manner that depends on the content of the stabilizer, and Nb₂O₅ and/or Ta₂O₅. The sintering time can be reduced, as long as the present invention can exhibit its effects. Shortening the sintering time can improve production efficiency and reduce energy costs.

In the method for producing the zirconia composite sintered body, the rate of temperature increase in sintering the molded body is not particularly limited, and is preferably 0.1°C/min or more, more preferably 0.2°C/min or more, even more preferably 0.5°C/min or more. The rate of temperature increase is preferably 50°C/min or less, more preferably 30°C/min or less, even more preferably 20°C/min or less. Productivity improves when the rate of temperature increase is at or above these lower limits.

A common dental zirconia furnace can be used for the sintering process of the molded body. The dental zirconia furnace may be a commercially available product. Examples of commercially available products include Noritake KATANA^{®} F-1, F-1N, F-2, F-2N (manufactured by SK Medical Electronics Co., Ltd.).

Preferably, the sintering process for the molded body includes a hot isostatic pressing (HIP) process, aside from sintering at the highest sintering temperature. The HIP process can further improve the translucency and strength of the zirconia composite sintered body.

In the following, the term "primary sintered body" is used to refer to sintered bodies obtained through sintering at the highest sintering temperature, whereas "HIP sintered body" is used to refer to sintered bodies after a HIP process.

Known hot isostatic pressing (HIP) devices can be used for the HIP process.

The temperature of the HIP process is not particularly limited. However, for advantages such as obtaining a high-strength and dense zirconia composite sintered body, the HIP temperature is preferably 1,200°C or more, more preferably 1,300°C or more, even more preferably 1,400°C or more. The HIP temperature is preferably 1,700°C or less, more preferably 1,650°C or less, even more preferably 1,600°C or less.

In the method for producing the zirconia composite sintered body, the pressure in the HIP process of the primary sintered body is not particularly limited. However, for advantages such as obtaining a high-strength and dense sintered body, the HIP pressure is preferably 100 MPa or more, more preferably 125 MPa or more, even more preferably 130 MPa or more. The upper limit of HIP pressure is not particularly limited, and may be, for example, 400 MPa or less, 300 MPa or less, or 200 MPa or less.

In the method for producing the zirconia composite sintered body, the rate of temperature increase in the HIP process of the primary sintered body is not particularly limited, and is preferably 0.1°C/min or more, more preferably 0.2°C/min or more, even more preferably 0.5°C/min or more. The rate of temperature increase is preferably 50°C/min or less, more preferably 30°C/min or less, even more preferably 20°C/min or less. Productivity improves when the rate of temperature increase is at or above these lower limits.

In the method for producing the zirconia composite sintered body used in the present invention, the duration of HIP in the HIP process of the primary sintered body is not particularly limited. However, for advantages such as obtaining a high-strength and dense zirconia composite sintered body, the duration of the HIP process is preferably 5 minutes or more, more preferably 10 minutes or more, even more preferably 30 minutes or more. The duration of the HIP process is preferably 10 hours or less, more preferably 6 hours or less, even more preferably 3 hours or less.

In the method for producing the zirconia composite sintered body, the pressure medium in the HIP process of the primary sintered body is not particularly limited. However, in view of a low impact on zirconia, the pressure medium may be at least one selected from the group consisting of oxygen gas, oxygen-mixed gas, air, and inert gas (for example, such as nitrogen gas, argon gas).

When conducting HIP processing of the primary sintered body in an oxygen-mixed gas atmosphere, the oxygen concentration may be, for example, more than 0% and 20% or less, though it is not particularly limited.

When using an oxygen-mixed gas, at least one inert gas (for example, such as nitrogen gas or argon gas) may be selected as a gas other than oxygen.

In the method for producing the zirconia composite sintered body, the HIP process may result in blackening due to oxygen deficiency when the process is carried out in a reducing atmosphere such as by using an inert gas. In order to prevent such blackening, it is preferable to include a heat treatment step (or tempering as it is also called hereinbelow), performed at 1,650°C or less in either the atmosphere or an excess oxygen atmosphere, after the HIP process. In view of the efficiency of heat treatment, the heat treatment step is carried out preferably in an excess oxygen atmosphere.

As used herein, "excess oxygen atmosphere" means an atmosphere where the oxygen concentration exceeds that of the atmosphere. The excess oxygen atmosphere is not particularly limited, as long as the oxygen concentration is higher than 21% and 100% or less, and can be appropriately selected within this range. For example, the oxygen concentration may be 100%.

The zirconia composite sintered body used in the present invention may be a primary sintered body, a HIP sintered body, or a tempered sintered body with no particular restriction, provided that the present invention can exhibit its effects.

A certain preferred embodiment is, for example, a zirconia composite sintered body that has been tempered.

The heat treatment temperature in the atmosphere or an excess oxygen atmosphere may be appropriately varied according the aesthetics of the zirconia composite sintered body (for example, the shade of dental prostheses).

In a certain preferred embodiment, in view of the aesthetics of the zirconia composite sintered body, the heat treatment temperature in the atmosphere or an excess oxygen atmosphere is preferably 1,650°C or less, more preferably 1,600°C or less, even more preferably 1,550°C or less.

In another preferred embodiment, in view of the aesthetics of the zirconia composite sintered body, the heat treatment temperature in the atmosphere or an excess oxygen atmosphere is preferably 1,400°C or less, more preferably 1,300°C or less, even more preferably 1,200°C or less.

In either embodiment, the heat treatment temperature is preferably 500°C or more, more preferably 600°C or more, even more preferably 700°C or more.

A common dental zirconia furnace can be used for the tempering process. The dental zirconia furnace may be a commercially available product. Examples of commercially available products include Noritake KATANA^{®} F-1, F-1N, F-2 (manufactured by SK Medical Electronics Co., Ltd.).

The zirconia composite sintered body used in the present invention exhibits excellent machinability despite being a sintered body. It is accordingly not necessary to machine it as a mill blank of a pre-sintered body in a semi-sintered state before sintering into a sintered body.

Nonetheless, the zirconia composite sintered body can be produced using a method whereby a molded body obtained from the raw material composition is pre-sintered to form a semi-sintered-state pre-sintered body, and this pre-sintered body, unprocessed, is machined before sintering it to form a sintered body.

Another example of the production method of the zirconia composite sintered body is a method for producing a zirconia composite sintered body that comprises the steps of fabricating a molded body using the raw material composition, pre-sintering the molded body to form a zirconia composite pre-sintered body (pre-sintering step), and sintering the zirconia composite pre-sintered body.

In order to ensure block formation, the firing temperature (pre-sintering temperature) in the pre-sintering step is, for example, preferably 800°C or more, more preferably 900°C or more, even more preferably 950°C or more.

The pre-sintering temperature is, for example, preferably 1,200°C or less, more preferably 1,150°C or less, even more preferably 1,100°C or less. The preferred range of pre-sintering temperatures is, for example, 800°C to 1,200°C. It is believed that, at these pre-sintering temperatures, no significant progression of the formation of the solid solution of the stabilizer occurs in the pre-sintering step.

The zirconia composite pre-sintered body has a density of preferably 2.7 g/cm³ or more. The zirconia composite pre-sintered body has a density of preferably 4.0 g/cm³ or less, more preferably 3.8 g/cm³ or less, even more preferably 3.6 g/cm³ or less. Processing becomes easier when the density falls within these ranges. The density of the composite pre-sintered body can be calculated, for example, as the mass-to-volume ratio of the composite pre-sintered body ((mass of composite pre-sintered body) / (volume of composite pre-sintered body)).

The zirconia composite pre-sintered body has a three-point flexural strength of preferably 15 to 70 MPa, more preferably 18 to 60 MPa, even more preferably 20 to 50 MPa.

The flexural strength can be measured in compliance with ISO 6872:2015 except for the specimen size, using a specimen measuring 5 mm in thickness, 10 mm in width, and 50 mm in length. For surface finishing, the specimen surfaces, including chamfered surfaces (45° chamfers at the corners of specimen), are finished longitudinally with #600 sandpaper. The specimen is disposed in such an orientation that its widest face is perpendicular to the vertical direction (loading direction). In the flexure test, measurements are made at a span of 30 mm with a crosshead speed of 1.0 mm/min.

The sintering step for the zirconia composite pre-sintered body can be conducted using the same method and conditions (such as temperature and pressure) used to sinter the molded body. Accordingly, in embodiments of the production method using a zirconia composite pre-sintered body, the term "molded body" can be read as referring to "pre-sintered body".

The zirconia composite sintered body used in the present invention excels in strength. The zirconia composite sintered body used in the present invention has a biaxial flexural strength of preferably 300 MPa or more, more preferably 350 MPa or more, even more preferably 400 MPa or more, yet more preferably 450 MPa or more, particularly preferably 500 MPa or more. The zirconia composite sintered body used in the present invention, with its biaxial flexural strength falling in these ranges, can reduce defects, for example, such as fracture in the oral cavity, when used as a dental prosthesis. The upper limit of biaxial flexural strength is not particularly limited, and may be, for example, 1,200 MPa or less, or 1,000 MPa or less. The biaxial flexural strength of the zirconia composite sintered body can be measured in compliance with ISO 6872:2015.

It is preferable that a zirconia composite sintered body of the present invention have high translucency. The translucency can be evaluated using ΔL*. Specifically, the zirconia composite sintered body used in the present invention has a translucency of preferably 10 or more, more preferably 12 or more, even more preferably 13 or more, particularly preferably 14 or more in terms of a ΔL* in a sample measuring 15 mm in diameter and 1.2 mm in thickness. A highly translucent zirconia composite sintered body can be obtained with ΔL* falling within these ranges.

ΔL* means the difference between the lightness (first L* value) against a white background and the lightness (second L* value) against a black background in the same sample. Specifically, ΔL* means the difference between the L* value against a white background (JIS Z 8781-4:2013 Color Measurements - Part 4: CIE 1976 L*a*b* color space) and the L* value against a black background. The white background means the white part of the hiding-power test paper described in JIS K 5600-4-1:1999, Part 4, Section 1, and the black background means the black part of the hiding-power test paper.

The upper limit of ΔL* is not particularly limited, and may be, for example, 25 or less. In view of aesthetics, ΔL* may be 20 or less.

A spectrophotometer can be used to measure ΔL* for a zirconia composite sintered body with a diameter of 15 mm and a thickness of 1.2 mm. For example, the dental colorimeter Crystaleye CE100-CE/JP with a 7-band LED illuminant and analysis software Crystaleye (manufactured by Olympus Corporation) may be used for measurement.

When the zirconia composite sintered body used in a method for producing a dental prosthesis of the present invention is one that comprises zirconia and a stabilizer capable of preventing a phase transformation of zirconia and in which the zirconia content is 78 to 97.5 mol%, the stabilizer content is 1 to 12 mol%, and the content of Nb₂O₅ and/or Ta₂O₅ is 1 to 9 mol% in total 100 mol% of zirconia, the stabilizer, Nb₂O₅, and Ta₂O₅, the zirconia composite sintered body can be produced using the same method used to produce the zirconia composite sintered body comprising a capping element or ion, except that the raw material composition does not contain a capping agent.

The zirconia composite sintered body can also be produced according to the method of production disclosed in WO2021/132644, depending on the content of each component and/or the crystal system of zirconia.

Examples of dental prostheses produced using the zirconia composite sintered body used in the present invention include crown restorations such as inlays, onlays, veneers, crowns, core-integrated crowns, and bridges, as well as abutment teeth, dental posts, dentures, denture bases, and implant parts (fixtures, abutments). Preferably, for example, a commercially available dental CAD/CAM system is used for machining. Examples of such CAD/CAM systems include the CEREC system manufactured by Dentsply Sirona, and the KATANA^{®} system manufactured by Kuraray Noritake Dental Inc.

The zirconia composite sintered body used in the present invention can also be used in applications other than dental use, and is particularly suited for zirconia parts that require irregular or complex shapes and strength.

Compared to sintered bodies produced solely by existing production methods (such as injection molding, CIP, casting, and 3D printing), the zirconia composite sintered body used in the present invention can be directly processed as a sintered body. This can be cost-effective, for example, when a desired zirconia part is obtained in short time periods. For parts that are complex in shape and difficult to produce with traditional methods, this technique eliminates the need for mechanical fitting of multiple parts, allowing for the production of zirconia parts with maintained high strength. Furthermore, because the sintered body is directly processable, the sintering process can be eliminated when precise dimensions are needed, eliminating uneven shrinkage during firing, leading to high-precision zirconia parts. The zirconia composite sintered body is also applicable to methods of manufacture in applications, specifically, such as in jewelry goods, engine parts and interior components of mobility vehicles such as aircraft and automobiles, display panel frames, construction members, electrical appliance components, components of household goods, and toy parts.

The zirconia parts can also be fitted with different materials to create composite components.

The present invention encompasses embodiments combining all or part of the foregoing features in various forms, provided that the present invention can exhibit its effects with such combinations within the technical idea of the present invention.

### EXAMPLES

The present invention is described below in greater detail through Examples. It is to be noted, however, that the present invention is in no way limited by the following Examples, and various modifications may be made by a person with ordinary skill in the art within the technical idea of the present invention.

### [Examples 1 to 20 and Comparative Examples 1 to 6]

The measurement samples for Examples and Comparative Examples were prepared through processes including the fabrication a granular raw material composition, a molded body, and a sintered body (the fabrication of a primary sintered body, as well as HIP processing and tempering).

### [Fabrication of Granular Raw Material Composition]

To fabricate a granular raw material composition for each Example and Comparative Example, a mixture was prepared by combining commercially available powders of ZrO₂, Y₂O₃, and at least one of Nb₂O₅ or Ta₂O₅, and optionally, a capping agent and a TiO₂ powder, so as to yield, after sintering, a zirconia composite sintered body with the content of each component indicated in Tables 1 to 3. Subsequently, water was added to prepare a slurry, and the slurry was pulverized by wet ball milling until it had an average particle diameter of 0.13 µm or less. After adding a binder to the pulverized slurry, the slurry was dried with a spray dryer to obtain a granular raw material composition (hereinafter, also referred to simply as "raw material composition"). This raw material composition was used to produce a molded body, as detailed below.

The average particle diameter was measured by volume with ultrasonic waves being applied after the slurry diluted with water was subjected to 30 minutes of ultrasonication, using a laser diffraction/scattering particle size distribution analyzer (Partica LA-950 manufactured by Horiba Ltd.).

### [Fabrication of Molded Body]

For each Example and Comparative Example, block-shaped molded bodies were produced in preparation for the fabrication of sintered body samples for the evaluation of surface roughness and glaze firing, as follows.

First, the raw material composition was placed in a mold with inside dimensions of 19 mm × 18 mm, ensuring that sintering produces a zirconia composite sintered body with a thickness of 14.5 mm.

Subsequently, the raw material composition was molded under a surface pressure of 200 MPa for 90 seconds to obtain a block-shaped molded body, using a uniaxial press molding machine.

### [Fabrication of Primary Sintered Body]

The block-shaped molded body was held for 2 hours in the atmosphere at a highest sintering temperature of 1,550°C, using the Noritake KATANA^{®} F-1 furnace manufactured by SK Medical Electronics Co., Ltd. This resulted in a block-shaped zirconia composite sintered body (primary sintered body) sample.

### [Fabrication of HIP Sintered Body]

The block-shaped zirconia composite sintered body (primary sintered body) was held for 2 hours in an argon atmosphere at 1,450°C under 150 MPa, using the HIP device O₂-Dr.HIP manufactured by Kobe Steel, Ltd. This resulted in a block-shaped zirconia composite sintered body (HIP sintered body) sample.

### [Fabrication of Zirconia Composite Sintered Body (Tempered Sintered Body)]

The block-shaped zirconia composite sintered body (HIP sintered body) was held at 700°C for 60 hours using the Noritake KATANA^{®} F-1 furnace (manufactured by SK Medical Electronics Co., Ltd.) to prepare a block-shaped zirconia composite sintered body (tempered sintered body) sample. The block-shaped sample measured 15.7 mm in width, 16.5 mm in length, and 14.5 mm in height.

### [Preparation of Desired Shapes Using Wet Processing Machine]

A metal fixture was attached to the surface of the block-shaped sample of the zirconia composite sintered body (tempered sintered body) of each Example (the surface measuring 15.7 mm in width and 14.5 mm in height). Using a CEREC system MC-XL (manufactured by Dentsply Sirona), the sintered body was then processed to prepare samples in anterior-tooth, premolar, and molar shapes for the evaluation of glaze firing.

For the machining program, inLab^{®} CAM version 20.0.1.203841 software was used with the following settings: Manufacture: IVOCLAR VIVADENT, Material name: IPS e.max CAD, Production Method: Grinding, Block size: C16. A Step Bur 12 and a Cylinder Pointed Bur 12S were used as the processing tools.

### [Pretreatment of Substrate (Zirconia Composite Sintered Body)]

For pretreatment of the machined sintered bodies prepared using the method described in the above [Preparation of Desired Shapes Using Wet Processing Machine], the machined sintered bodies of Examples 1 to 7, 12, 13, 15, and 17 to 19 were subjected to heat treatment as indicated in Table 1.

The machined sintered bodies were polished in Examples 8 to 11, 14, 16 and 20. The following dental polishers were used for polishing: EVE Diapol Twist Medium (manufactured by EVE) in Example 8; Pearl Surface Z (manufactured by Kuraray Noritake Dental Inc.) in Examples 9, 11, 14, 16 and 20; and Meister Cone S-Green (manufactured by Kuraray Noritake Dental Inc.) in Example 10.

### [Measurement Method for Surface Roughness Ra of Sintered Body]

The pretreated crown-shaped sintered bodies of Examples 8 to 11, 14, 16 and 20 were evaluated for surface roughness Ra, specifically as follows.

For each crown-shaped sintered body, the surface was examined using a color 3D laser microscope (VK-9710 manufactured by Keyence Corporation under this trade name) under the following conditions.
Measurement pitch: 2 µm
Measurement distance: 1,058 µm

The acquired image was analyzed with image analysis software (VK Analyzer manufactured by Keyence Corporation under this trade name) to determine surface roughness, and the surface roughness Ra was calculated (arithmetic mean value for n = 3).

### [Evaluation of Glaze Firing]

The pretreated sintered bodies of Examples and the as-processed sintered bodies of Comparative Examples were fired after building up the porcelains listed in Tables 1 to 3, as follows.

First, the porcelain was coated or sprayed over the crown-shaped sintered body following the procedures specified in the instructions or other manuals provided by each manufacturer. Here, the porcelain was applied so as to cover the entire substrate surface, without leaving any portion of the surface exposed. In addition, the porcelain was built up to ensure that the thickness becomes 10 to 50 µm after firing. This was followed by firing according to the firing program specified in the instructions or other manuals provided by the manufacturers of the respective porcelains, using a dental furnace (Cerafusion BX, manufactured by SK Medical Electronics Co., Ltd. under this trade name). The surface of the resulting evaluation sample was then examined to determine whether there is defective fusing. The results are presented in Tables 1 to 3.

The following criteria were used for the evaluation of glaze firing.
P: No delamination of the glaze porcelain from the substrate was observed in both the frontal and occlusal aspects.
F: Delamination of the glaze porcelain from the substrate was observed in the frontal or occlusal aspect.

Here, "P" indicates pass, and "F" indicates fail.

The content of each component in the sintered bodies presented in Tables 1 to 3 was calculated from the quantities of the raw materials charged.

The content (mol%) of capping elements or ions (e.g., Na) in Tables 1 to 3 refers to the proportion external to total 100 mol% of zirconia (ZrO₂ and HfO₂ combined), the stabilizer (yttria), Nb₂O₅, and Ta₂O₅.

The content (mol%) of zirconia (ZrO₂ and HfO₂ combined), the stabilizer, Nb₂O₅, and Ta₂O₅ in Tables 1 to 3 refers to the content of each component in total 100 mol% of zirconia, the stabilizer, Nb₂O₅, and Ta₂O₅.

The content (mass%) of TiO₂ and Al₂O₃ in Tables 1 to 3 refers to the proportion external to total 100 mass% of zirconia, the stabilizer, Nb₂O₅, and Ta₂O₅.

A/B in Tables 1 to 3 represents the ratio of A to B, where A is the Y₂O₃ content in mol%, and B is the combined content of Nb₂O₅ and Ta₂O₅ in mol%.

**[Table 1]**

| | Substrate | | | | | | | | | | |
|---|---|---|---|---|---|---|---|---|---|---|---|
| | Composition | | | Content of components | | | | | | | |
| | ZrO2, stabilizer (Y2O3), Nb2O5 or Ta2O5 | Capping agent | Optional component | Unit [mol%] | | | | | Element or ion derived from capping agent [mol%] | Optional component (TiO2 or Al2O3) [mass%] | A/B |
| | | | | (1) ZrO2 and HfO2 | (2) Y2O3 (A) | (3) Nb2O5 (B) | (4) Ta2O5 (B) | (1) to (4) in total | | | |
| Ex. 1 | ZrO2, Y2O3, Nb2O5 | NaOH | TiO2 | 90.30 | 5.30 | 4.40 | 0.00 | 100.00 | 0.18 | 3.00 | 1.20 |
| Ex. 2 | ZrO2, Y2O3, Nb2O5 | NaOH | TiO2 | 90.30 | 5.30 | 4.40 | 0.00 | 100.00 | 0.18 | 3.00 | 1.20 |
| Ex. 3 | ZrO2, Y2O3, Nb2O5 | NaOH | TiO2 | 90.30 | 5.30 | 4.40 | 0.00 | 100.00 | 0.18 | 3.00 | 1.20 |
| Ex. 4 | ZrO2, Y2O3, Nb2O5 | NaOH | TiO2 | 90.30 | 5.30 | 4.40 | 0.00 | 100.00 | 0.18 | 3.00 | 1.20 |
| Ex. 5 | ZrO2, Y2O3, Nb2O5 | NaOH | TiO2 | 90.30 | 5.30 | 4.40 | 0.00 | 100.00 | 0.18 | 3.00 | 1.20 |
| Ex. 6 | ZrO2, Y2O3, Nb2O5 | - | - | 90.30 | 5.30 | 4.40 | 0.00 | 100.00 | - | - | 1.20 |
| Ex. 7 | ZrO2, Y2O3, Nb2O5 | - | - | 90.30 | 5.30 | 4.40 | 0.00 | 100.00 | - | - | 1.20 |
| Ex. 8 | ZrO2, Y2O3, Nb2O5 | NaOH | TiO2 | 90.30 | 5.30 | 4.40 | 0.00 | 100.00 | 0.18 | 3.00 | 1.20 |
| Ex. 9 | ZrO2, Y2O3, Nb2O5 | NaOH | TiO2 | 90.30 | 5.30 | 4.40 | 0.00 | 100.00 | 0.18 | 3.00 | 1.20 |
| Ex. 10 | ZrO2, Y2O3, Nb2O5 | NaOH | TiO2 | 90.30 | 5.30 | 4.40 | 0.00 | 100.00 | 0.18 | 3.00 | 1.20 |

**[Table 1] Continued**

| | Pretreatment | | Porcelain (Trade name, manufacturer) | Outcome of glaze firing | | |
|---|---|---|---|---|---|---|
| | Heat treatment | Polishing | | Anterior tooth | Premolar | Molar |
| Ex. 1 | 300°C × 1 min | - | FC Paste Stain (Kuraray Noritake Dental Inc.) | P | P | P |
| Ex. 2 | 1000°C × 5 min | - | FC Paste Stain (Kuraray Noritake Dental Inc.) | P | P | P |
| Ex. 3 | 300°C × 1 min | - | CZR Press Glaze (Kuraray Noritake Dental Inc.) | P | P | P |
| Ex. 4 | 300°C × 30 sec | - | FC Paste Stain (Kuraray Noritake Dental Inc.) | P | P | F |
| Ex. 5 | 300°C × 10 sec | - | FC Paste Stain (Kuraray Noritake Dental Inc.) | P | F | F |
| Ex. 6 | 1000°C × 5 min | - | FC Paste Stain (Kuraray Noritake Dental Inc.) | P | P | P |
| Ex. 7 | 300°C × 1 min | - | FC Paste Stain (Kuraray Noritake Dental Inc.) | P | P | P |
| Ex. 8 | - | Ra = 4.17 µm | FC Paste Stain (Kuraray Noritake Dental Inc.) | P | P | P |
| Ex. 9 | - | Ra = 1.60 µm | FC Paste Stain (Kuraray Noritake Dental Inc.) | P | P | P |
| Ex.10 | - | Ra = 7.39 µm | FC Paste Stain (Kuraray Noritake Dental Inc.) | P | P | F |

| | | | | | | |
|---|---|---|---|---|---|---|
| P: pass, F: fail | | | | | | |

**[Table 2]**

| | Substrate | | | | | | | | | | |
|---|---|---|---|---|---|---|---|---|---|---|---|
| | Composition | | | Content of components | | | | | | | |
| | ZrO2, stabilizer (Y2O3), Nb2O5 or Ta2O5 | Capping agent | Optional component | Unit [mol%] | | | | | Element or ion derived from capping agent [mol%] | Optional component (TiO2 or Al2O3) [mass%] | A/B |
| | | | | (1) ZrO2 and HfO2 | (2) Y2O3 (A) | (3) Nb2O5 (B) | (4) Ta2O5 (B) | (1) to (4) in total | | | |
| Ex. 11 | ZrO2, Y2O3, Nb2O5 | NaOH | TiO2 | 90.30 | 5.30 | 4.40 | 0.00 | 100.00 | 0.18 | 3.00 | 1.20 |
| Ex. 12 | ZrO2, Y2O3, Nb2O5 | NaOH | TiO2 | 90.30 | 5.30 | 4.40 | 0.00 | 100.00 | 0.18 | 3.00 | 1.20 |
| Ex. 13 | ZrO2, Y2O3, Nb2O5 | NaOH | TiO2 | 90.30 | 5.30 | 4.40 | 0.00 | 100.00 | 0.18 | 3.00 | 1.20 |
| Ex. 14 | ZrO2, Y2O3, Nb2O5 | NaOH | TiO2 | 90.30 | 5.30 | 4.40 | 0.00 | 100.00 | 0.18 | 3.00 | 1.20 |
| Ex. 15 | ZrO2, Y2O3, Nb2O5 | NaOH | TiO2 | 90.30 | 5.30 | 4.40 | 0.00 | 100.00 | 0.18 | 3.00 | 1.20 |
| Ex. 16 | ZrO2, Y2O3, Nb2O5 | NaOH | TiO2 | 90.30 | 5.30 | 4.40 | 0.00 | 100.00 | 0.18 | 3.00 | 1.20 |
| Ex. 17 | ZrO2, Y2O3, Nb2O5 | NaOH | TiO2 | 90.30 | 5.30 | 4.40 | 0.00 | 100.00 | 0.18 | 3.00 | 1.20 |
| Ex. 18 | ZrO2, Y2O3, Nb2O5 | NaOH | TiO2 | 90.30 | 5.30 | 4.40 | 0.00 | 100.00 | 0.18 | 3.00 | 1.20 |
| Ex. 19 | ZrO2, Y2O3, Nb2O5 | NaOH | TiO2 | 90.30 | 5.30 | 4.40 | 0.00 | 100.00 | 0.18 | 3.00 | 1.20 |
| Ex. 20 | ZrO2, Y2O3, Nb2O5 | - | - | 90.30 | 5.30 | 4.40 | 0.00 | 100.00 | - | - | 1.20 |

**[Table 2] Continued**

| | Pretreatment | | Porcelain (Trade name, manufacturer) | Outcome of glaze firing | | |
|---|---|---|---|---|---|---|
| | Heat treatment | Polishing | | Anterior tooth | Premolar | Molar |
| Ex.11 | - | Ra = 1.42 µm | CZR Press Glaze (Kuraray Noritake Dental Inc.) | P | P | P |
| Ex.12 | 300°C × 1 min | - | Universal Spray Glaze (Dentsply Sirona Inc.) | P | P | P |
| Ex.13 | 1000°C × 5 min | - | Universal Spray Glaze (Dentsply Sirona Inc.) | P | P | P |
| Ex.14 | - | Ra = 1.57 µm | Universal Spray Glaze (Dentsply Sirona Inc.) | P | P | P |
| Ex.15 | 300°C × 1 min | - | Akzent Plus Glaze SPRAY (VITA Inc.) | P | P | P |
| Ex.16 | - | Ra = 1.45 µm | Akzent Plus Glaze SPRAY (VITA Inc.) | P | P | P |
| Ex.17 | 300°C × 1 min | - | Crystal Glaze Spray (Ivoclar Vivadent Inc.) | P | P | P |
| Ex.18 | 1000°C × 5 min | - | Crystall./ Glaze Spray (Ivoclar Vivadent Inc.) | P | P | P |
| Ex.19 | 300°C × 1 min | - | TANAKA Z Luster (ATD JAPAN Co., Ltd. ) | P | P | P |
| Ex.20 | - | Ra = 1.55 µm | FC Paste Stain (Kuraray Noritake Dental Inc.) | P | P | P |

| | | | | | | |
|---|---|---|---|---|---|---|
| P: pass, F: fail | | | | | | |

**[Table 3]**

| | Substrate | | | | | | | | | | |
|---|---|---|---|---|---|---|---|---|---|---|---|
| | Composition | | | Content of components | | | | | | | |
| | ZrO2, stabilizer (Y2O3), Nb2O5 or Ta2O5 | Capping agent | Optional component | Unit [mol%] | | | | | Element or ion derived from capping agent [mol%] | Optional component (TiO2 or Al2O3) [mass%] | A/B |
| | | | | (1) ZrO2 and HfO2 | (2) Y2O3 (A) | (3) Nb2O5 (B) | (4) Ta2O5 (B) | (1) to (4) in total | | | |
| Com. Ex. 1 | ZrO2, Y2O3, Nb2O5 | NaOH | TiO2 | 90.30 | 5.30 | 4.40 | 0.00 | 100.00 | 0.18 | 3.00 | 1.20 |
| Com. Ex. 2 | ZrO2, Y2O3, Nb2O5 | NaOH | TiO2 | 90.30 | 5.30 | 4.40 | 0.00 | 100.00 | 0.18 | 3.00 | 1.20 |
| Com. Ex. 3 | ZrO2, Y2O3, Nb2O5 | NaOH | TiO2 | 90.30 | 5.30 | 4.40 | 0.00 | 100.00 | 0.18 | 3.00 | 1.20 |
| Com. Ex. 4 | ZrO2, Y2O3, Nb2O5 | NaOH | TiO2 | 90.30 | 5.30 | 4.40 | 0.00 | 100.00 | 0.18 | 3.00 | 1.20 |
| Com. Ex. 5 | ZrO2, Y2O3, Nb2O5 | NaOH | TiO2 | 90.30 | 5.30 | 4.40 | 0.00 | 100.00 | 0.18 | 3.00 | 1.20 |
| Com. Ex. 6 | ZrO2, Y2O3, Nb2O5 | NaOH | TiO2 | 90.30 | 5.30 | 4.40 | 0.00 | 100.00 | 0.18 | 3.00 | 1.20 |

**[Table 3] Continued**

| | Pretreatment | | Porcelain (Trade name, manufacturer) | Outcome of glaze firing | | |
|---|---|---|---|---|---|---|
| | Heat treatment | Polishing | | Anterior tooth | Premolar | Molar |
| Com. Ex. 1 | - | - | FC Paste Stain (Kuraray Noritake Dental Inc.) | F | F | F |
| Com. Ex. 2 | - | - | CZR Press Glaze (Kuraray Noritake Dental Inc.) | F | F | F |
| Com. Ex. 3 | - | - | Universal Spray Glaze (Dentsply Sirona Inc.) | F | F | F |
| Com. Ex. 4 | - | - | Akzent Plus Glaze SPRAY (VITA Inc.) | F | F | F |
| Com. Ex. 5 | - | - | Crystal Glaze Spray (Ivoclar Vivadent Inc.) | F | F | F |
| Com. Ex. 6 | - | - | TANAKA Z Luster (ATD JAPAN Co., Ltd. ) | F | F | F |

| | | | | | | |
|---|---|---|---|---|---|---|
| P: pass, F: fail | | | | | | |

As can be seen from these results, the zirconia composite sintered bodies according to Examples 1 to 20, as shown in FIG. 2A or 2B, exhibited excellent aesthetics in the dental prostheses by enabling favorable fusing of porcelain not only as anterior teeth or premolars but also as prostheses having regions complex in shape and including a number of curved surfaces along with multiple grooves seen in the occlusal aspect, such as molars.

In contrast, the zirconia composite sintered bodies according to Comparative Examples 1 to 6 were found to be inferior in aesthetics, with the glaze porcelain delaminating from the substrate in the frontal or occlusal aspect, as indicated in the areas encircled in black in FIG. 3A or 3B.

### INDUSTRIAL APPLICABILITY

The zirconia composite sintered body used in the present invention exhibits excellent machinability while possessing suitable strength and translucency. This makes the zirconia composite sintered body particularly useful as a dental prosthesis or other dental materials intended for dental treatment.

## Claims

1. A method for producing a dental prosthesis, comprising the steps of:
subjecting a zirconia composite sintered body comprising Nb₂O₅ and/or Ta₂O₅ to heat treatment or polishing as a pretreatment; and
building up porcelain on the zirconia composite sintered body subjected to the pretreatment, followed by firing.

2. The method for producing a dental prosthesis according to claim 1, wherein the heat treatment is carried out at a temperature of 150°C or more and 1,550°C or less.

3. The method for producing a dental prosthesis according to claim 1, wherein the zirconia composite sintered body has a surface roughness Ra of 8.0 µm or less after being polished in the polishing step.

4. The method for producing a dental prosthesis according to claim 1 or 2, wherein the zirconia composite sintered body comprises zirconia, and a stabilizer capable of preventing a phase transformation of zirconia, and
the zirconia content is 78 to 97.5 mol%, the content of the stabilizer is 1 to 12 mol%, and the content of Nb₂O₅ and/or Ta₂O₅ is 1 to 9 mol% in total 100 mol% of zirconia, the stabilizer, Nb₂O₅, and Ta₂O₅.

5. The method for producing a dental prosthesis according to claim 4, wherein the zirconia composite sintered body further comprises an element or ion derived from a capping agent.

6. The method for producing a dental prosthesis according to claim 5, wherein the content of the element or ion derived from a capping agent is more than 0 mol% and 5 mol% or less relative to total 100 mol% of zirconia, the stabilizer, Nb₂O₅, and Ta₂O₅.

7. The method for producing a dental prosthesis according to claim 5, wherein the element or ion derived from a capping agent belongs to periods 2 to 7 of the periodic table, and is an element or an ion thereof having a first ionization energy lower than that of a group 18 element of the same period, and/or an element or an ion thereof having a high electron affinity.

8. The method for producing a dental prosthesis according to claim 5, wherein the element or ion derived from a capping agent comprises at least one element or ion thereof selected from the group consisting of Cu, Ag, Li, Na, K, Rb, Cs, Fr, At, I, Br, Cl, and F.

9. The method for producing a dental prosthesis according to claim 5, wherein the element or ion derived from a capping agent comprises at least one element or ion thereof selected from the group consisting of Li, Na, K, Rb, Cs, and Fr.

10. The method for producing a dental prosthesis according to claim 4, wherein the zirconia composite sintered body has a ratio A/B of 0.9 or more and 3 or less, where A is the content of the stabilizer in mol%, and B is the combined content of Nb₂O₅ and Ta₂O₅ in mol%.

11. The method for producing a dental prosthesis according to claim 4, wherein the stabilizer comprises Y₂O₃ and/or CeO₂.

12. The method for producing a dental prosthesis according to claim 4, wherein the zirconia composite sintered body further comprises a zirconia enhancer, and the content of the zirconia enhancer is more than 0 mass% and 6.0 mass% or less relative to total 100 mass% of zirconia, the stabilizer, and Nb₂O₅ and Ta₂O₅.

13. The method for producing a dental prosthesis according to claim 12, wherein the zirconia enhancer comprises TiO₂ and/or Al₂O₃.

14. The method for producing a dental prosthesis according to claim 13, wherein the zirconia enhancer comprises TiO₂, and the content of TiO₂ is 0.6 to 4.5 mass%.

15. The method for producing a dental prosthesis according to claim 1 or 2, wherein the zirconia composite sintered body has an average crystal grain size of 0.5 to 5.0 µm.
